# EUROPÄISCHE PATENTANMELDUNG

(11) **EP 0 881 238 A2**
(43) Veröffentlichungstag der Anmeldung: **02.12.1998**
(21) Anmeldenummer: 98109075.6
(22) Anmeldetag: 19.05.1998
(51) Int. Cl.: C08F 20/04, C08F 251/00, A61L 15/60, A61L 15/24

(54) **Wasserquellbare, hydrophile Polymerzusammensetzungen**

(30) Priorität: 28.05.1997 DE 19722340
(71) Anmelder: Clariant GmbH, 65929 Frankfurt am Main (DE)
(72) Erfinder: Engelhardt, Fritz, Dr., Chesapeake, Virginia 23320 (US); Funk, Rüdiger, Dr., 65527 Niedernhausen (DE); Herfert, Norbert, Dr., 63674 Altenstadt (DE); Weismantel, Matthias, 63637 Jossgrund-Oberndorf (DE)

(57) **Zusammenfassung**

Die vorliegende Erfindung betrifft wasserquellbare, hydrophile Polymerzusammensetzungen, herstellbar durch radikalische (Co)polymerisation von einem oder mehreren hydrophilen Monomeren in Anwesenheit von Stärke und/oder chemisch modifizierter Stärke, dadurch gekennzeichnet, daß ein Radikalinitiator eingesetzt wird, der drei oder mehr Radikalstellen pro Molekül ausbildet.

## Beschreibung

Die Erfindung betrifft wasserquellbare, hydrophile Polymerzusammensetzungen aus einer Kombination von synthetischen Polymeren und Stärke oder chemisch modifizierten Stärkederivaten, Verfahren zu ihrer Herstellung und ihre Verwendung zur Herstellung von Hygieneartikeln, wie Windeln, Damenbinden und Inkontinenzartikeln.

Die meisten der heute verwendeten Absorptionsmaterialien, auch Superabsorber genannt, die in der Lage sind, in kurzer Zeit große Flüssigkeitsmengen wie Wasser oder Urin aufzunehmen, stellen in erster Linie schwach vernetzte Polyacrylate dar. Diese Absorptionsmaterialien basieren somit nicht auf nachwachsenden Rohstoffen und sind nur unzureichend bzw. überhaupt nicht biologisch abbaubar.

Im Zuge des gesteigerten Umweltbewußtseins gibt es Bestrebungen, Superabsorber ganz oder teilweise auf nachwachsenden Rohstoffen aufzubauen. Geeignete nachwachsende Rohstoffe stellen die Polysaccharide dar, wobei ein ganz besonders geeigneter nachwachsender Rohstoff Stärke ist. Diese steht in ausreichend großen Mengen zur Verfügung und ist, ebenso wie chemisch modifizierte Stärke-Derivate, außerdem auch leicht biologisch abbaubar.

Superabsorber auf reiner Stärke-Basis sind bereits bekannt. So wird in US 5.079.354 ein Absorptionsmaterial basierend auf vernetzter Carboxymethylstärke beschrieben. Aus der EP-A 637594 ist ein wasserabsorbierendes Hart aus einem vernetzten Polysaccharid bekannt, wobei letzteres eine Carboxyalkylcellulose oder eine Carboxyalkylstärke, die durch Reaktion mit einer Aminosäure vernetzt wird, sein kann. In der DE-A 44 42 605 werden quellbare Stärkeester beansprucht, die durch teilweise Veresterung von Stärke oder modifizierter Stärke mit einem Carbonsäureanhydrid oder einer Mischung von Carbonsäureanhydriden und Vernetzung erhältlich sind. In der DE-A 44 42 606 wird die Herstellung von Superabsorbermaterial durch Umsetzung von Stärke oder modifizierter Stärke mit einem oder mehreren Carbonsäureanhydriden, bevorzugt Maleinsäureanhydrid, beschrieben. Alle bisher bekannten Superabsorber-Materialien auf reiner Stärke-Basis weisen jedoch im Vergleich zu den marktüblichen Superabsorbern auf Polyacrylat-Basis eine deutlich geringere Absorptionskapazität für wäßrige Flüssigkeiten auf, insbesondere unter Druckbelastung. Superabsorber-Materialien auf reiner Stärke-Basis können daher in den heute verwendeten Windelkonstruktionen Polyacrylat-Superabsorber nur unter Inkaufnahme von größeren und letztlich inakzeptablen Performance-Verlusten der Windel ersetzen.

Im Bestreben, die Absorptionskapazität der reinen Polyacrylat-Superabsorber beizubehalten, aber den Anteil an aus nachwachsenden Rohstoffen aufgebauten Bestandteilen zu erhöhen, sind zahlreiche Absorptionsmaterialien aus Polyacrylat und Polysaccharid hergestellt worden.
DE-C 26 12 846 beschreibt Pfropfpolymere von Acrylsäure auf Polysaccharide, wie z. B. Maisstärke. Dabei können allerdings nur geringe Mengen an Polysacchariden (bis max. 25 %) eingesetzt werden, da sich ansonsten die Absorptionseigenschaften drastisch verschlechtern.
Genauso können durch Einarbeitung von Polysacchariden ins Polymerisationsgel von Polyacrylaten, wie in DE-A 40 29 591, DE-A 40 29 592 und DE-A 40 29 593 beschrieben, die Polyacrylate nur bis zu max. 25 % ersetzt werden, ohne eine deutliche Verschlechterung des Aufnahmevermögens und anderer Eigenschaften der resultierenden Superabsorber zu erhalten, auch wenn zusätzlich diverse Hilfsstoffe, wie Fasern und z. B. Aluminiumvernetzer zugesetzt werden. Die DE-C 31 32 976 beschreibt die Vermischung von Polyacrylsäure mit Polysacchariden, bevorzugt unvernetzte Carboxymethylcellulose, in Pulverform und in Lösung. Allerdings werden nach den hierhin beschriebenen Verfahren keinerlei Bindungen zwischen Polyacrylsäure- und Polysaccharid-Komponente erhalten, so daß die Polysaccharid-Komponente wieder leicht aus dem Absorptionsmaterial extrahiert werden kann, somit nichts mehr zu der Absorptionskapazität beiträgt und im Gegenteil die Flüssigkeitsaufnahme durch Erhöhung der Viskosität der die Superabsorber-Partikel umgebenden Lösung erschwert.

WO94/25519 und WO94/25520 beschreiben Polymerzusammensetzungen bestehend aus wasserlöslichen und/oder wasserquellbaren Polymeren auf Basis von Polysacchariden und wasserquellbare synthetische Polymere, wobei diese Polymere durch wenigstens eine mindestens bifunktionelle Verbindung vernetzt sind. Allerdings benötigen diese Polymerzusammensetzungen noch die Zusätze von Matrixmaterialien, ionischen oder kovalenten Vernetzern und Antiblockingmitteln zur Verhinderung der Entmischung, des Gelblocking und des Zusammenbackens an feuchter Luft. Durch diese Zusätze wird jedoch die Absorptionskapazität auf unerwünschte Weise verringert.

Der vorliegenden Erfindung liegt die Aufgabe zugrunde, auf einfachem Wege eine wasserquellbare, hydrophile Polymerzusammensetzung basierend auf einem synthetischen Polymer und Stärke oder einem chemisch modifizierten Stärke-Derivat bereitzustellen, die die zuvor beschriebenen Nachteile nicht aufweist und folgende Eigenschaften besitzt:
a) Sie soll einen möglichst hohen Stärke-Anteil aufweisen, so daß sie grundsätzlich zumindest teilweise biologisch abbaubar ist.
b) Sie soll eine möglichst hohe Aufnahmekapazität für Wasser und wäßrige Flüssigkeiten besitzen, d.h. die Stärke- bzw. Stärkederivat-Komponente sollte einen aktiven Beitrag zum Absorptionsvermögen leisten.
c) Sie sollte einen möglichst geringen Anteil an extrahierbaren Bestandteilen aufweisen, d.h. die Stärke- bzw. Stärkederivat-Polymerketten sollten mit den Polymerketten des synthetischen Polymers zu einem Netzwerk verbunden sein.

Es wurde nun überraschend gefunden, daß diese Aufgabe gelöst werden kann durch Polymerisation hydrophiler Monomerer in Anwesenheit von Stärke bzw. Stärkederivaten unter Verwendung von Radikalinitiatoren, die drei oder mehr Radikalstellen pro Molekül ausbilden können. Die Verwendung solcher Radikalinitiatoren für die Herstellung von Superabsorbern ist bereits in der EP-A 675142 beschrieben worden. Allerdings kann diesem Dokument keine Lehre zur Verbesserung der Performance von Produkten mit hohem Anteil an Stärke bzw. Stärkederivaten entnommen werden.

Erfindungsgemäß erfolgt die Lösung der erfindungsgemäßen Aufgabe somit durch eine wasserquellbare, hydrophile Polymerzusammensetzung, herstellbar durch radikalische (Co)polymerisation von einem oder mehreren hydrophilen Monomeren in Anwesenheit von Stärke und/oder chemisch modifizierter Stärke, dadurch gekennzeichnet, daß ein Radikalinitiator eingesetzt wird, der drei oder mehr Radikalstellen pro Molekül ausbildet.

Das Gewichtsverhältnis zwischen synthetischer, d.h. aus hydrophilen Monomeren erhaltener, Polymerkomponente und Stärke- bzw. Stärkederivat-Komponente beträgt insbesondere 90:10 bis 10:90, bevorzugt 70:30 bis 20:80, und besonders bevorzugt 60:40 bis 30:70.

Geeignete hydrophile Monomere sind beispielsweise polymerisationsfähige Säuren, wie Acrylsäure, Methacrylsäure, Capronsäure, Vinylsulfonsäure, Vinylphosphonsäure, Maleinsäure einschließlich dessen Anhydrid, Fumarsäure, Itaconsäure, 2-Acrylamido-2-methylpropansulfonsäure sowie deren Amide, Hydroxyalkylester und aminogruppen- oder ammoniumgruppenhaltiger Ester und Amide. Desweiteren wasserlösliche N-Vinylamide oder auch Diallyldimethylammoniumchlorid.

Bevorzugte hydrophile Monomere sind Verbindungen der allgemeinen Formel I 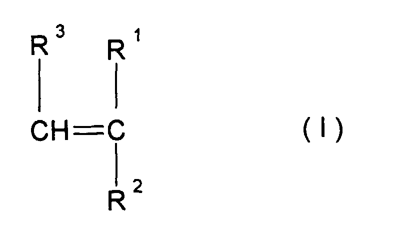 worin
- R¹: Wasserstoff, Methyl oder Ethyl,
- R²: die Gruppe -COOR⁴, die Sulfonylgruppe, die Phosphonylgruppe, die mit (C₁-C₄)-Alkanol veresterte Phosphonylgruppe oder eine Gruppe der Formel 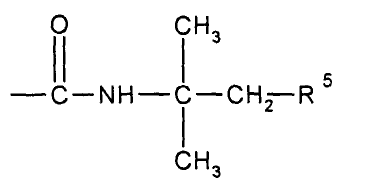
- R³: Wasserstoff, Methyl, Ethyl oder die Carboxylgruppe,
- R⁴: Wasserstoff, Amino oder Hydroxy-(C₁-C₄)-alkyl und
- R⁵: die Sulfonylgruppe, die Phosphonylgruppe oder die Carboxylgruppe
bedeuten.

Besonders bevorzugte hydrophile Monomere sind Acrylsäure und Methacrylsäure.

Geeignete Stärken sind prinzipiell alle aus natürlichen Vorkommen generierbare Stärken. Beispiele sind native oder vorverkleisterte Mais-Stärke, native oder vorverkleisterte Wachs-Mais-Stärke, native oder vorverkleisterte Kartoffelstärke, native oder vorverkleisterte Weizen-Stärke, native oder vorverkleisterte Amylo-Mais-Stärke oder native oder vorverkleisterte Tapioka-Stärke. Besonders bevorzugt sind vorverkleisterte Mais-Stärke und vorverkleisterte Kartoffel-Stärke.

Geeignete chemisch modifizierte Stärken sind beispielsweise säurekatalytisch, enzymatisch oder thermisch abgebaute Stärken, oxidierte Stärken, Stärkeether wie z. B. Allylstärke oder Hydroxyalkylstärken wie 2-Hydroxyethylstärken, 2-Hydroxypropylstärken oder 2-Hydroxy-3-trimethyl-ammoniopropylstärken, oder Carboxyalkylstärken wie Carboxymethylstärken, Stärkeester wie z. B. Monocarbonsäureester der Stärke wie Stärkeformiate, Stärkeacetate, Stärkeacrylat, Stärkemethacrylat oder Stärkebenzoate, Stärkeester von Di- und Polycarbonsäuren wie Stärkesuccinate oder Stärkemaleate, Stärkecarbamidsäureester (Stärkeurethane), Stärkedithiokohlensäureester (Stärkexanthogenate), oder Stärkeester anorganischer Säuren wie Stärkesulfate, Stärkenitrate oder Stärkephosphate, Stärkeesterether wie z.B. 2-Hydroxyalkylstärkeacetate, oder Vollacetale der Stärke, wie sie z. B. bei der Umsetzung von Stärke mit aliphatischen oder cyclischen Vinylether gebildet werden. Besonders bevorzugt sind Carboxymethylstärken, Stärkesuccinate oder Stärkemaleate.

Als Radikalinitiatoren können im Prinzip alle Verbindungen eingesetzt werden, die mit oder ohne Einwirkung zusätzlicher Aktivatoren wie Licht, Strahlung, Wärme, Ultraschall, Redoxmittel usw., drei oder mehr Radikalstellen pro Molekül ausbilden. Dies bedeutet, daß diese Radikalinitiatoren drei, vier oder mehr Gruppen enthalten, die Radikale bilden können. Die Radikalstellen können dabei gleichzeitig gebildet werden, in der Regel werden sie aber zeitlich versetzt, d. h. nacheinander gebildet. Geeignet sind beispielsweise Verbindungen, die mindestens drei Hydroperoxid-Einheiten, Peroxid-Einheiten oder Azo-Einheiten enthalten.

Geeignet sind beispielsweise Polyhydroperoxide, die durch anodische Oxidation von Polycarbonsäuren, insbesondere von Polyacrylsäure und Polymethacrylsäure in Gegenwart von Sauerstoff erhalten werden können (J. Pol. Sci. Vol. XXXIV, Seiten 287 bis 307 (1959)).

Peroxid-Einheiten können beispielsweise als Percarbonat, Perketal- oder Perester-Einheiten vorliegen. Beispiele für solche Verbindungen sind insbesondere Dioxetanverbindungen und tert.-Butylperester, wie beispielsweise Methacrylat-tert.-Butylperacrylat-Copolymere (J. Pol. Sci. Vol. XXXIV, Seite 301 (1959)).

Im übrigen werden in 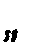 The Chemistry of Functional Groups, Peroxides", edited by S. Patai 1983, John Wiley & Sons Ltd., Chapter 13, by Ray Ceresa geeignete Verbindungen mit mehreren Peroxid- bzw. Hydroperoxid-Einheiten und Synthesen beschrieben. Der Inhalt dieser Veröffentlichung ist ausdrücklich Bestandteil vorliegender Offenbarung.

Es ist bevorzugt, Hydroperoxid- bzw. Peroxid-Einheiten enthaltende Radikalinitiatoren in Verbindung mit Reduktionsmitteln einzusetzen. Geeignete Reduktionsmittel sind beispielsweise Fe²⁺, Ascorbinsäure, Sulfinsäuren, Sulfite sowie Formamidinsulfinsäuren und deren Salze.

Geeignete Verbindungen, die drei oder mehr Azo-Einheiten enthalten, sind beispielsweise Umsetzungsprodukte von
a) Azodicarbonsäuren mit Verbindungen, die mehr als zwei Oxiran-Funktionen enthalten. Je nach verwendeter Oxiranverbindung können auf diese Weise Tri- bis Oligoverbindungen und Polymere erhalten werden.
   Eine bevorzugte Azodicarbonsäure ist insbesondere 4,4'-Azobis(4-cyanovaleriansäure), die beispielsweise mit Polyglycerinpolyglycidylethern geeignete Radikalinitiatoren bildet.
b) hydroxyl- und aminofunktionellen Azoverbindungen mit Verbindungen, die mehr als zwei Oxiran- Gruppen enthalten.
   Geeignete Azoverbindungen sind beispielsweise 2,2'-Azobis(N,N-dimethylenisobutyramidin) oder das entsprechende Dihydrochlorid, 2,2'-Azobis(2-amidinopropan)-dihydrochlorid, 2,2'-Azobis(2-methyl-N-(1,1-bis(hydroxymethyl)-2-hydroxyethyl)-propionamid), 2,2'-Azobis(2-methyl-N-(1,1-bis(hydroxymethyl)-ethyl)propionamid) oder 2,2'-Azobis-(2-methyl-N-(2-hydroxyethyl)-propionamid), die beispielsweise mit dem oben unter a) genannten Polyglycidylether geeignete Radikalinitiatoren bilden.
c) Azobisnitrilen mit Tri- oder Polyalkoholen. Bevorzugt sind insbesondere Umsetzungsprodukte von 2,2'-Azobisisobutyronitril mit Glycerin, Trimethylolpropan, Threit, Erythrit, Pentaerrythrit, Arabit, Adonit, Xylit, Sorbit, Mannit oder Dulcit.

Die genannten Radikalinitiatoren können allein oder auch in beliebigen Mischungen untereinander zur Herstellung der erfindungsgemäßen wasserquellbaren, hydrophilen Polymerzusammensetzung verwendet werden.

Dabei werden sie bevorzugt in Mengen von 0,001 bis 20 Gew.-%, bezogen auf die Gesamtmonomeren eingesetzt. Besonders bevorzugt sind 0,05 bis 3,0 Gew.-%.

In einer besonderen Ausführungsform der vorliegenden Erfindung werden Radikalinitiatoren verwendet, deren Radikale bildende Funktionen unterschiedliche Reaktivitäten besitzen bzw. durch unterschiedliche Mechanismen aktiviert werden. Solche Initiatoren enthalten also beispielsweise sowohl Azo-, als auch Peroxid- oder Hydroperoxidfunktionen, die in einer vorbestimmten Weise nacheinander aktiviert werden und so beispielsweise zur Herstellung von Blockpolymeren verwendet werden können.
Es kann darüber hinaus von Vorteil sein, Initiatoren zu verwenden, deren radikalbildende Funktionen in unterschiedlichen räumlichen Abständen voneinander in dem Molekül liegen.

Das Molekulargewicht der zur Herstellung erfindungsgemäßer Polymerzusammensetzungen verwendbarer Initiatoren kann naturgemäß in weiten Grenzen schwanken. Die Molekulargewichte liegen insbesondere im Bereich von 100 bis 10 000 000.

Die erfindungsgemäßen Polymerzusammensetzungen können auch unter Verwendung geeigneter Vernetzer, d. h. von Verbindungen mit mindestens zwei Doppelbindungen, die in das Netzwerk der synthetischen Polymerkomponente einpolymerisiert werden können, hergestellt sein.

Geeignete Vernetzer sind insbesondere Methylenbisacryl- bzw. -methacrylamid, Ester ungesättigter Mono- oder Polycarbonsäuren von Polyolen, wie Diacrylat oder Triacrylat, z. B. Butandiol- oder Ethylenglykoldiacrylat bzw.- methacrylat, Trimethylolpropantriacrylat sowie Vinylmethacrylat und Allylverbindungen wie Allyl(meth)acrylat, Triallylcyanurat, Maleinsäurediallylester, Polyallylester, Allylether von Polyolen, wie z. B. Pentaerythritoldi- und -triallylether, Tetraallyloxyethan, Triallylamin, Tetraallylethylendiamin, Allylester der Phosphorsäure sowie Vinylphosphonsäurederivate, wie sie beispielsweise in der EP-A 343427 beschrieben sind. Der Inhalt der EP-A 343427 ist ausdrücklich auch Bestandteil der vorliegenden Offenbarung.

Der Vernetzeranteil liegt bevorzugt bei 0 bis 20 Gew.-%, besonders bevorzugt bei 0 bis 3 Gew.-%, bezogen auf den Gesamtmonomerenanteil.

Darüber hinaus können die erfindungsgemäßen Polymerzusammensetzungen in an sich bekannter Weise in wäßriger Gelphase nachvernetzt und/oder als gemahlene und abgesiebte Polymerpartikel oberflächenvernetzt sein. Dazu geeignete Vernetzer sind Verbindungen, die mindestens zwei Gruppen enthalten, die mit den Carboxylgruppen und/oder den Hydroxylgruppen der Polymerzusammensetzung kovalente Bindungen bilden können. Geeignete Verbindungen sind beispielsweise Di- oder Polyglycidylverbindungen, wie Phosphonsäurediglycidylester, Alkoxysilylverbindungen, Polyaziridine, Polyamine, Polyamidoamine und deren Umsetzungsprodukte mit Epichlorhydrin, Di- oder Polyalkohole, Divinylsulfon oder Di- oder Polyaldehyde wie z. B. Glyoxal. Besonders geeignete Vernetzer sind Phosphonsäurediglycidylether, wie in EP-A 481370 und EP-A 543303 beschrieben, und Polyamidoamin-Epichlorhydrin-Addukte, wie sie insbesondere in der EP-A 349935 beschrieben sind. Der Inhalt der vorstehend genannten Patentanmeldungen ist ausdrücklich Bestandteil auch der vorliegenden Offenbarung.

Die erfindungsgemäßen wasserquellbaren, hydrophilen Polymerzusammensetzungen können durch bekannte Polymerisationsverfahren hergestellt werden, z. B. durch Polymerisation in wäßriger Phase nach dem Verfahren der inversen Suspensionspolymerisation. Besonders bevorzugt ist jedoch die Polymerisation in wäßriger Lösung nach dem Verfahren der sogenannten Gelpolymerisation. Dabei werden auf Feststoff bezogen 15 bis 60 Gew.-% wäßrige Lösungen in Gegenwart eines Radikalinitiators, der Tri- oder Polyradikale bilden kann, bevorzugt ohne mechanische Durchmischung unter Ausnutzung des Trommsdorff-Norrish-Effektes (Bios Final Rep. 363.22; Makromol. Chem. 1, 169 (1947)), polymerisiert.

Die Polymerisationsreaktion kann im Temperaturbereich zwischen 0°C und 130°C, vorzugsweise zwischen 10 und 100°C, sowohl bei Normaldruck als auch unter erhöhtem Druck, durchgeführt werden. Wie üblich kann die Polymerisation auch in einer Schutzgasatmosphäre, vorzugsweise unter Stickstoff, ausgeführt werden. Durch mehrstündiges Nachheizen der wäßrigen Polymerisatgele im Temperaturbereich von 50 bis 130°C, vorzugsweise 70 bis 100°C, können die Qualitätseigenschaften der Polymerisate noch verbessert werden.

Die auf diesem Wege hergestellten, in Form wäßriger Gallerten vorliegenden erfindungsgemäßen Polymerzusammensetzungen können nach mechanischer Zerkleinerung mit geeigneten Apparaten durch bekannte Trocknungsverfahren in fester Form erhalten werden und zum Einsatz gelangen. Ein besonders bevorzugtes Trocknungsverfahren stellt hierbei das Verfahren der Walzentrocknung dar, das eine produktschonende Kurzzeittrocknung ermöglicht.

Die erfindungsgemäßen wasserquellbaren, hydrophilen Polymerzusammensetzungen basieren teilweise auf nachwachsenden Rohstoffen, sind zum Teil biologisch abbaubar und weisen im Vergleich zu bekannten Stärke/Polyacrylat-Polymerzusammensetzungen des Standes der Technik deutliche Vorteile auf. Insbesondere weisen sie einen niedrigeren Anteil an extrahierbaren Bestandteilen, eine stärkere Anbindung der Stärke-Polymerketten an das Polyacrylat-Netzwerk und einen höheren Beitrag der Stärke-Polymerkomponente zu den Quelleigenschaften auf. Sie haben daher ein hohes Flüssigkeitsbindevermögen bei gleichzeitig hohen Flüssigkeitsretentionswerten und hoher mechanischer Festigkeit gequollener Gelpartikel sowie hoher Permeabilität von gequollenen Gelschichten. Sie sind deshalb in hervorragender Weise als Absorptionsmittel für Wasser und wäßrige Flüssigkeiten, wie Urin oder Blut, in Hygieneartikeln wie Baby- und Erwachsenenwindeln, Binden, Tampons und dergleichen geeignet. Sie können aber auch als Bodenverbesserungsmittel in Landwirtschaft und Gartenbau, als Feuchtigkeitsbindemittel bei der Kabelummantelung sowie zum Eindicken wäßriger Abfälle verwendet werden.

### Beschreibung der in den Beispielen verwendeten Testmethoden:

### FSC (Free Swell Capacity):

Zur Bestimmung der FSC werden 0,2 g absorbierendes Produkt (SAP) (Kornfraktion 106 - 850 µm) in einen 60 x 60 mm großen Teebeutel eingewogen, der anschließend verschweißt wird. Der Teebeutel wird dann in einen Überschuß von 0,9 Gew.-%iger Kochsalz-Lösung gegeben (mindestens 1,25 l Kochsalz-Lösung /1 g SAP). Nach 20 Minuten Quellzeit wird der Teebeutel aus der Kochsalz-Lösung genommen und die überschüssige Lösung wird 10 Minuten lang abtropfen lassen. Danach wird durch Wägung des Teebeutels die von dem SAP absorbierte Flüssigkeitsmenge ermittelt.

### CRC (Centrifuge Retention Capacity):

Zur Bestimmung der CRC werden 0,2 g SAP (Kornfraktion 106 - 850 µm) in einen 60 x 60 mm großen Teebeutel eingewogen, der anschließend verschweißt wird. Der Teebeutel wird dann in einen Überschuß von 0,9 Gew.-%iger Kochsalz-Lösung gegeben (mindestens 1,25 l Kochsalz-Lösung/1 g SAP). Nach 20 Minuten Quellzeit wird der Teebeutel aus der Kochsalz-Lösung genommen und bei 250 g drei Minuten lang zentrifugiert. Durch Wägung des zentrifugierten Teebeutels wird die von dem SAP festgehaltene Flüssigkeitsmenge ermittelt.

### Extrahierbare Bestandteile:

Zur Bestimmung der extrahierbaren Bestandteile werden 1 g SAP (Kornfraktion 106 - 850 µm) in 200 g 0,9 Gew.-%iger Kochsalzlösung eingerührt. Die Mischung wird 1 Stunde lang gerührt und anschließend filtriert. Ein Aliquot des Filtrats wird im Trockenschrank bei ca. 105-110°C bei schwachem Luftdurchsatz bis zur Gewichtskonstanz getrocknet. Durch Wägung des Rückstandes können die extrahierbaren Bestandteile unter Berücksichtigung des Kochsalz-Gehaltes der Lösung ermittelt werden.

### AUL (Absorbency under Load):

Die Absorption unter Druck (AUL) wurde in bekannter Weise, wie beispielsweise in der EP-A 339 461 beschrieben bestimmt. Die AUL 20 bezieht sich hierbei auf die Messung der Absorption unter einer Druckbelastung von 20 g/cm², die AUL 40 bezieht sich auf die Messung der Absorption unter einer Druckbelastung von 40 g/cm², wobei die Flächenbelegung der Superabsorber-Partikel (Kornfraktion 300 - 600 µm) in der Meßzelle 0,032 g/cm² beträgt.

### Herstellung von Radikalinitiatoren:

a) Herstellung eines polyfunktionellen Azoinitiators:
   Apparatur: Glasautoklav mit Rührer, Innenthermometer, HCl-Einlaßventil und Druckmanometer.
   Zu 33,5 g (0,25 mol) Trimethylolpropan in 300 ml trockenem Chloroform wurden 164,2 g (1,0 mol) 2,2'-Azobisisobutyronitril in 400 ml Chloroform gegeben. Unter Rühren wurde die Reaktionsmischung auf 2°C abgekühlt. HCl-Gas wurde in den Autoklaven eingeleitet, und nach der Absättigung der Lösung mit dem Gas wurde ein HCl-Überdruck von 4 bar eingestellt. Nach 48 h bei 2°C wurde der HCl-Überdruck abgelassen und die Reaktionsmischung in 600 ml Eiswasser gegeben. Die organische Phase wurde abgetrennt, mit gesättigter NaCl-Lösung gewaschen und mit gesättigter NaHCO₃-Lösung entsäuert. Die organische Phase wurde anschließend über Na₂SO₄ getrocknet und das Lösungsmittel im Ölpumpenvakkum bei Raumtemperatur abdestilliert. Isoliert wurde ein wachsartig festes gelbes Produkt.
b) Herstellung eines polyfunktionellen Radikalinitiators:
   Apparatur: Doppelmantelbecherglas-Elektrolysezelle mit seitlichem Schliffansatz, Teflonstopfen mit Bohrungen für Elektroden, Gaseinleitungsrohr und Thermometer; Pt-Blech-Elektroden auf Halterung; Kryostat; Galvanostat mit Stromzuleitungen, Meßgeräten, etc.
   150 g einer wäßrigen Lösung, die 8,3 % (12,45 g, 0,173 Mol-Äqiv. COOH) Polyacrylsäure (M_{w} = ca. 200000) enthielt, wurde mit 0,35 g NaOH (0,0086 Mol) versetzt, in die Elektrolysezelle überführt und mit Hilfe eines Kryostaten auf 10°C temperiert. Über das Gaseinleitungsrohr, an dessen unteres Ende eine Glasfritte angeschmolzen war, wurde nun ein steter O₂-Strom in die Lösung eingeleitet. Man elektrolysierte unter Rühren bei einem Strom von 150 mA bis zu einem Ladungsdurchsatz von 1800 C, wobei die Innentemperatur bei 10°C gehalten und der Elektrolyt ständig mit Sauerstoff gespült wurde. Das Elektrolysat wurde in dieser Form unmittelbar für Polymerisationsversuche eingesetzt.

### Vergleichsbeispiel 1

In einem durch geschäumtes Kunststoffmaterial gut isoliertes Polyethylengefäß mit einem Fassungsvermögen von 10 l werden 2220 g E-Wasser vorgelegt, 752,5 g Natriumbicarbonat darin suspendiert und langsam 990 g Acrylsäure so zudosiert, daß ein Überschäumen der Reaktionslösung vermieden wird, wobei sich diese auf eine Temperatur von ca. 5 bis 3°C abkühlt. Es werden nun 4 g Trimethylolpropantriacrylat und eine Lösung von 1210 g der in Tabelle 1 angegebenen Stärken bzw. Stärke-Derivate in 3020 g gekühltem Wasser zugegeben. Durch diese Lösung wird Stickstoff durchgeleitet, bis ein Restsauerstoffgehalt von 2 ppm erreicht wird. Bei einer Temperatur von 4°C werden die Initiatoren nacheinander zugegeben und gut verrührt. Verwendet wird ein thermischer Initiator, 2,6 g 2,2'-Azobisamidinopropan-dihydrochlorid, gelöst in 20 g E-Wasser, und ein Redox-Initiatorsystem, 0,8 g Kaliumpersulfat, gelöst in 170 g E-Wasser, sowie 0,4 g Ascorbinsäure, gelöst in 120 g E-Wasser. Die Reaktionslösung wird daraufhin ohne Rühren stehengelassen, wobei durch einsetzende Polymerisation, in dessen Verlauf die Temperatur bis auf ca. 60°C ansteigt, ein festes Gel entsteht. Dieses wird anschließend mechanisch zerkleinert, mit Hilfe eines Walzentrockners getrocknet, gemahlen und auf 100 bis 850 µm abgesiebt. Die Produkte weisen folgende physikalische Daten auf:

**Tabelle 1**

| Stärkederivat | FSC | CRC | Extrahierbare |
|---|---|---|---|
| EMOX D 30 S | 29 g/g | 21 g/g | 30,1 % |
| EMCOL H7 | 31 g/g | 22 g/g | 28,2 % |
| C* PUR 01915 | 32 g/g | 21 g/g | 35,4 % |
| Primojel | 33 g/g | 23 g/g | 31,8 % |
| Succinat-Stärke (DS = 0,05) | 32 g/g | 23 g/g | 29,5 % |
| Solviton N | 27 g/g | 18 g/g | 25,4 % |

- EMOX D 30 S: oxidierte Stärke der Fa. Emsland-Stärke
- EMCOL H7:: Hydroxypropyl-Stärke der Fa. Emsland-Stärke
- C^{*} PUR 01915:: enzymatisch abgebaute Stärke
- Primojel:: Carboxymethylstärke der Fa. AVEBE
- Solviton N:: 2-Hydroxy-3-trimethylammoniopropylstärke der Fa. AVEBE

Die erhaltenen Produkte wurden in einem weiteren Schritt durch Oberflächenvernetzung modifiziert. Hierzu wurden jeweils 50 g Produkt in einem Küchen-Mixer mit einer Lösung aus 0,05 g Methylphosphonsäurediglycidylester, 0,05 g Nonaethylenglykoldiglycidylether und 1,9 g Wasser vermischt. Die Produkte wurden anschließend im Trockenschrank 60 Minuten bei 120°C nachgetempert. Es wurden folgende physikalischen Daten erhalten:

**Tabelle 2**

| Stärkederivat | CRC | AUL 20 | AUL 40 |
|---|---|---|---|
| EMOX D 30 S | 18 g/g | 16 g/g | 10 g/g |
| EMCOL H7 | 17 g/g | 16 g/g | 11 g/g |
| C* PUR 01915 | 17 g/g | 14 g/g | 8 g/g |
| Primojel | 18 g/g | 17 g/g | 11 g/g |
| Succinat-Stärke (DS = 0,05) | 18 g/g | 17 g/g | 10 g/g |
| Solviton N | 14 g/g | 12 g/g | 9 g/g |

### Beispiel 1

Es wird wie im Vergleichsbeispiel angegeben verfahren, als thermischer Initiator wird jedoch anstelle von 2,2'-Azobisamidinopropan-dihydrochlorid 2,9 g des wie vorstehend unter a) beschrieben erhaltenen polyfunktionellen Radikalinitiators, als Redoxinitiatorsystem wird anstelle von Kaliumpersulfat/Ascorbinsäure 60 g des wie vorstehend unter b) beschrieben erhaltenen Elektrolysats, sowie 0,4 g Ascorbinsäure, gelöst in 120 g E-Wasser, eingesetzt. Tabelle 3 zeigt die erhaltenen physikalischen Daten der Produkte vor Oberflächenvernetzung:

**Tabelle 3**

| Stärkederivat | FSC | CRC | Extrahierbare |
|---|---|---|---|
| EMOX D 30 S | 30 g/g | 21 g/g | 20,1 % |
| EMCOL H7 | 31 g/g | 23 g/g | 19,3 % |
| C* PUR 01915 | 32 g/g | 22 g/g | 22,7 % |
| Primojel | 34 g/g | 24 g/g | 21,5 % |
| Succinat-Stärke (DS = 0,05) | 31 g/g | 23 g/g | 19,0 % |
| Solviton N | 25 g/g | 19 g/g | 16,4 % |

Wie ein Vergleich der Tabellen 1 und 3 zeigt, werden bei Einsatz des polyfunktionellen Radikalinitiators Produkte mit geringerem Gehalt an Extrahierbaren erhalten.

Die erhaltenen Produkte wurden analog zum Vergleichsbeispiel durch Oberflächenvernetzung modifiziert. Es wurden folgende physikalischen Daten erhalten:

**Tabelle 4**

| Stärkederivat | CRC | AUL 20 | AUL 40 |
|---|---|---|---|
| EMOX D 30 S | 18 g/g | 17 g/g | 12 g/g |
| EMCOL H7 | 18 g/g | 18 g/g | 12 g/g |
| C* PUR 01915 | 17 g/g | 16 g/g | 10 g/g |
| Primojel | 19 g/g | 19 g/g | 13 g/g |
| Succinat-Stärke (DS = 0,05) | 18 g/g | 19 g/g | 13 g/g |
| Solviton N | 15 g/g | 14 g/g | 11 g/g |

Wie ein Vergleich der Tabellen 2 und 4 zeigt, werden bei Verwendung des polyfunktionellen Radikalinitiators Produkte mit höheren AUL 20- und AUL 40-Werten erhalten.

### Vergleichsbeispiel 2

Unter adiabatischen Bedingungen werden in einem 5 l zylindrischen Weithalsreaktionskolben 558 g Acrylsäure, 1,8 g Tetraallyloxyethan und 292 g der in Tabelle 5 angegebenen Stärken vorgelegt. In einem zweiten Reaktionskolben werden 4060 g auf 15°C abgekühltes E-Wasser vorgelegt und Stickstoff eingeleitet. Bei einem Sauerstoffgehalt von ca. 1,5 ppm werden 10 g einer 4 %igen wäßrigen Lösung von 2,2'-Azobis(2-Amidinopropan)-dihydrochlorid zugegeben, nach weiterem Einleiten von Stickstoff und einem Sauerstoff-Gehalt von ca. 1,3 ppm werden 8 g einer 0,75 %igen Wasserstoffperoxid-Lösung zugegeben und schließlich bei einem Sauerstoff-Gehalt von < 1,0 ppm werden 8 g einer 0,15 %igen Ascorbinsäurelösung zugegeben. Danach wird diese Lösung zu der Mischung aus Acrylsäure, Tetraallyloxyethan und Stärke im ersten Weithalsreaktionskolben überführt. Durch einsetzende Polymerisation, in deren Verlauf die Temperatur bis auf ca. 60°C ansteigt, entsteht ein festes Gel, das anschließend mechanisch zerkleinert wird. 1000 g des zerkleinerten Gels werden mit 270 g Natronlauge 27 %ig versetzt (Neutralisationsgrad der Acrylsäure = 78 Mol-%), dreimal durchgeknetet, und anschließend mit Hilfe eines Walzentrockners getrocknet, gemahlen und auf 100 bis 850 µm abgesiebt. Die Produkte weisen folgende physikalische Daten auf:

**Tabelle 5**

| Stärke | FSC | CRC | Extrahierbare |
|---|---|---|---|
| EMJEL E 30 | 48 g/g | 42 g/g | 24,9 % |
| Primojel | 49 g/g | 43 g/g | 25,5 % |
| EMJEL PG | 45 g/g | 39 g/g | 24,5 % |
| Cerestar AJ 12014 | 45 g/g | 40 g/g | 23,6 % |
| Farinex WM 85 | 41 g/g | 35 g/g | 29,7 % |
| Cerestar Pt 20002 | 45 g/g | 39 g/g | 23,7 % |
| Tapioka starch Full Past I | 50 g/g | 45 g/g | 28,9 % |

- EMJEL E 30:: kaltwasserlösliche Kartoffelstärke der Fa. Emsland-Stärke
- Primojel:: Carboxymethyl-Stärke der Fa. AVEBE
- EMJEL PG:: Phosphat-Stärke der Fa. Emsland-Stärke
- Cerestar AJ 12014:: kaltwasserlösliche Maisstärke der Fa. Cerestar
- Farinex WM 85:: kaltwasserlösliche Waxy-Maisstärke
- Cerestar Pt 20002:: native Weizenstärke der Fa. Cerestar
- Tapioka starch Full Past I:: kaltwasserlösliche Tapioka-Stärke

Die erhaltenen Produkte wurden durch Oberflächenvernetzung modifiziert. Hierzu wurden jeweils 50 g Produkt in einem Küchen-Mixer mit einer Lösung aus 0,01 g n-Propylphosphonsäurediglycidylester, 0,09 g Monoethylenglykoldiglycidylether, 2,0 g Wasser und 2,0 g i-Propanol vermischt. Die Produkte wurden anschließend im Trockenschrank 60 Minuten bei 120°C nachgetempert. Es wurden folgende physikalischen Daten erhalten:

**Tabelle 6**

| Stärkederivat | CRC | AUL 20 | AUL 40 |
|---|---|---|---|
| EMJEL E 30 | 32 g/g | 15 g/g | 12 g/g |
| Primojel | 32 g/g | 24 g/g | 15 g/g |
| EMJEL PG | 30 g/g | 23 g/g | 11 g/g |
| Cerestar AJ 12014 | 34 g/g | 25 g/g | 13 g/g |
| Farinex WM 85 | 25 g/g | 16 g/g | 10 g/g |
| Cerestar Pt 20002 | 35 g/g | 19 g/g | 12 g/g |
| Tapioka starch Full Past I | 32 g/g | 14 g/g | 9 g/g |

### Beispiel 2

Es wird wie in Vergleichsbeispiel 2 verfahren, als Redoxinitiatorsystem wird jedoch anstelle von Wasserstoffperoxid/Ascorbinsäure 40 g des wie vorstehend unter b) beschrieben erhaltenen Elektrolysats sowie 8 g einer 0,15 %igen Ascorbinsäurelösung eingesetzt. Tabelle 7 zeigt die erhaltenen physikalischen Daten der Produkte vor Oberflächenvernetzung:

**Tabelle 7**

| Stärkederivat | FSC | CRC | Extrahierbare |
|---|---|---|---|
| EMJEL E 30 | 48 g/g | 43 g/g | 13,5 % |
| Primojel | 49 g/g | 45 g/g | 13,4 % |
| EMJEL PG | 47 g/g | 40 g/g | 14,0 % |
| Cerestar AJ 12014 | 47 g/g | 43 g/g | 12,4 % |
| Farinex WM 85 | 41 g/g | 34 g/g | 21,5 % |
| Cerestar Pt 20002 | 46 g/g | 41 g/g | 16,8 % |
| Tapioka starch Full Past I | 50 g/g | 44 g/g | 19,2 % |

Wie ein Vergleich der Tabellen 5 und 7 zeigt, werden bei Einsatz des polyfunktionellen Radikalinitiators Produkte mit geringerem Gehalt an extrahierbaren Bestandteilen erhalten.

Die erhaltenen Produkte wurden analog zum Vergleichsbeispiel 2 durch Oberflächenvernetzung modifiziert. Es wurden folgende physikalischen Daten erhalten:

**Tabelle 8**

| Stärkederivat | CRC | AUL 20 | AUL 40 |
|---|---|---|---|
| EMJEL E 30 | 31 g/g | 26 g/g | 14 g/g |
| Primojel | 32 g/g | 30 g/g | 23 g/g |
| EMJEL PG | 30 g/g | 27 g/g | 14 g/g |
| Cerestar AJ 12014 | 33 g/g | 28 g/g | 15 g/g |
| Farinex WM 85 | 25 g/g | 24 g/g | 13 g/g |
| Cerestar Pt 20002 | 31 g/g | 25 g/g | 14 g/g |
| Tapioka starch Full Past I | 30 g/g | 22 g/g | 12 g/g |

Wie ein Vergleich der Tabellen 6 und 8 zeigt, werden bei Verwendung des polyfunktionellen Radikalinitiators Produkte mit höheren AUL 20- und AUL 40-Werten erhalten.

### Vergleichsbeispiel 3

In einem 2 l-Polymerisationskolben werden 635 g Cyclohexan vorgelegt und unter Rühren auf 40 bis 45 °C erwärmt, worauf die Zugabe von 3,5 g Ethylcellulose (Typ CN 200 der Fa. HERCULES, USA) erfolgt. Unter Einleiten eines schwachen Stickstoff-Stromes wird auf Rückflußtemperatur erhitzt. Nach 25 Minuten Rückfluß wird mittels einer Dosierpumpe eine auf Raumtemperatur abgekühlte Lösung, bestehend aus 245 g Wasser, 69 g Acrylsäure, 77,4 g Kalilauge 50 %ig, 161 g der in Tabelle 9 angegebenen Stärken bzw. Stärkederivaten, abgemischt mit einer Lösung aus 20 g Wasser, 0,15 g Ethylendiamintetraessigsäure, 0,05 g Kaliumpersulfat und 0,1 g 4,4'-Azobis-4-cyano-valeriansäure innerhalb von 90 Minuten zudosiert. Der Rückflußkühler wird nun gegen einen Wasserabscheider ausgetauscht und das Wasser wird azeotrop abdestilliert. Nach Beginn des azeotropen Abdestillierens des Wassers wird eine Emulsion, bestehend aus 15 g Cyclohexan, 0,5 g Wasser. 0,25 g Stearylphosphonsäurediglycidylester, 0,025 g Ethylenglykoldiglycidylether und 0,4 g Sorbitanmonolaurat, zugegeben. Es werden 318 g Wasser abdestilliert, das Lösungsmittel vom Polymer abfiltriert, 2 Stunden bei 105°C im Trockenschrank nachgetrocknet und gegebenfalls auf 100 bis 850 µm abgesiebt. Die Produkte weisen folgende physikalische Daten auf:

**Tabelle 9**

| Stärkederivat | FSC | CRC | AUL 20 | AUL 40 | Extrahierbare |
|---|---|---|---|---|---|
| Allylstärke (DS= 0,005) | 24 g/g | 15 g/g | 12 g/g | 8 g/g | 36 % |
| Stärkemaleat (DS = 0,05) | 26 g/g | 16 g/g | 13 g/g | 9 g/g | 42 % |
| Stärkesuccinat (DS = 0,02) | 25 g/g | 15 g/g | 13 g/g | 9 g/g | 45 % |
| Primojel | 27 g/g | 17 g/g | 14 g/g | 10 g/g | 39 % |
| EMOX D 30 S | 22 g/g | 13 g/g | 11 g/g | 8 g/g | 47 % |
| EMCOL H7 | 24 g/g | 14 g/g | 12 g/g | 8 g/g | 43 % |
| EMJEL PG | 22 g/g | 13 g/g | 11 g/g | 8 g/g | 41 % |

- Primojel:: Carboxymethyl-Stärke der Fa. AVEBE
- EMOX D 30 S:: oxidierte Stärke der Fa. Emsland-Stärke
- EMCOL H7:: Hydroxypropyl-Stärke der Fa. Emsland-Stärke
- EMJEL PG:: Phosphat-Stärke der Fa. Emsland-Stärke

### Beispiel 3

Es wird wie in Vergleichsbeispiel 3 verfahren, als Initiator wird anstelle von Kaliumpersulfat und 4,4'-Azobis-4-cyano-valeriansäure jedoch 0,35 g des wie in a) oben berschrieben erhaltenen polyfunktionellen Azoinitiators eingesetzt. Tabelle 10 zeigt die erhaltenen physikalischen Daten der Produkte:

**Tabelle 10**

| Stärkederivat | FSC | CRC | AUL 20 | AUL 40 | Extrahierbare |
|---|---|---|---|---|---|
| Allylstärke (DS = 0,005) | 22 g/g | 14 g/g | 15 g/g | 12 g/g | 23 % |
| Stärkemaleat (DS = 0,05) | 26 g/g | 16 g/g | 16 g/g | 12 g/g | 24 % |
| Stärkesuccinat (DS = 0,02) | 25 g/g | 16 g/g | 16 g/g | 13 g/g | 22 % |
| Primojel | 28 g/g | 18 g/g | 18 g/g | 14 g/g | 20 % |
| EMOX D 30 S | 23 g/g | 12 g/g | 13 g/g | 11 g/g | 24 % |
| EMCOL H7 | 24 g/g | 14 g/g | 15 g/g | 12 g/g | 23 % |
| EMJEL PG | 23 g/g | 13 g/g | 15 g/g | 12 g/g | 22 % |

Wie ein Vergleich der Tabellen 9 und 10 zeigt, werden bei Verwendung des polyfunktionellen Radikalinitiators Produkte mit niedrigeren Extrahierbaren und höheren AUL 20- und AUL 40-Werten erhalten.

## Patentansprüche

1. Wasserquellbare hydrophile Polymerzusammensetzung, herstellbar durch radikalische (Co)polymerisation von einem oder mehreren hydrophilen Monomeren in Anwesenheit von Stärke und/oder chemisch modifizierter Stärke, dadurch gekennzeichnet, daß ein Radikalinitiator eingesetzt wird, der drei oder mehr Radikalstellen pro Molekül ausbildet.

2. Wasserquellbare hydrophile Polymerzusammensetzung gemäß Anspruch 1, dadurch gekennzeichnet, daß hydrophile Monomere Verbindungen der allgemeinen Formel I 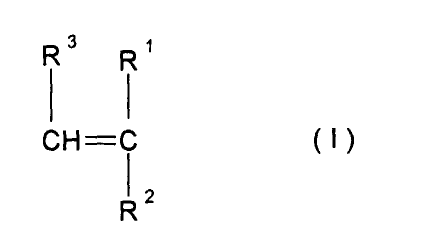 worin
R¹ Wasserstoff, Methyl oder Ethyl,
R² die Gruppe -COOR⁴, die Sulfonylgruppe, die Phosphonylgruppe, die mit (C₁-C₄)-Alkanol veresterte Phosphonylgruppe oder eine Gruppe der Formel 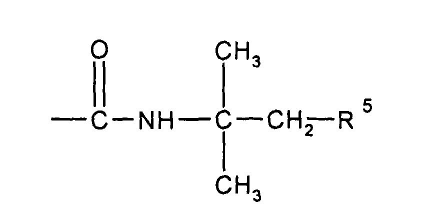
R³ Wasserstoff, Methyl, Ethyl oder die Carboxylgruppe,
R⁴ Wasserstoff, Amino oder Hydroxy-(C₁-C₄)-alkyl und
R⁵ die Sulfonylgruppe, die Phosphonylgruppe oder die Carboxylgruppe
bedeuten, sind.

3. Wasserquellbare hydrophile Polymerzusammensetzung gemäß Anspruch 1 und/oder 2, dadurch gekennzeichnet, daß als Stärke native oder vorverkleisterte Mais-Stärke, native oder vorverkleisterte Wachs-Mais-Stärke, native oder vorverkleisterte Kartoffelstärke, native oder vorverkleisterte Weizen-Stärke, native oder vorverkleisterte Amylo-Mais-Stärke, native oder vorverkleisterte Tapioka-Stärke und/oder als chemisch modifizierte Stärke säurekatalytisch, enzymatisch oder thermisch abgebaute Stärken, oxidierte Stärken, Stärkeether wie Allylstärke oder Hydroxyalkylstärken wie 2-Hydroxyethylstärken, 2-Hydroxypropyl-stärken und 2-Hydroxy-3-trimethyl-ammoniopropylstärken, Carboxyalkylstärken wie Carboxymethylstärken, Stärkeester wie Stärkeformate, Stärkeacetate, Stärkeacrylat, Stärkemethacrylat und Stärkebenzoate, Stärkeester wie Stärke-succinate und Stärkemaleate, Stärkecarbamidsäureester (Stärkeurethane), Stärkedithiokohlensäureester (Stärkexanthogenate), Stärkeester anorganischer Säuren wie Stärkesulfate, Stärkenitrate und Stärkephosphate, Stärkeesterether wie 2-Hydroxyalkylstärkeacetate, und Vollacetale der Stärke, wie die Umsetzungsprodukte von Stärke mit aliphatischen oder cyclischen Vinylether, eingesetzt wird.

4. Wasserquellbare hydrophile Polymerzusammensetzung gemäß einem oder mehreren der Ansprüche 1 bis 3, dadurch gekennzeichnet, daß als Stärke vorverkleisterte Mais-Stärke oder vorverleisterte Kartoffel-Stärke und/oder als chemisch modifizierte Stärke Carboxymethylstärke, Stärkesuccinat oder Stärkemaleat eingesetzt wird.

5. Wasserquellbare hydrophile Polymerzusammensetzung gemäß einem oder mehreren der Ansprüche 1 bis 4, dadurch gekennzeichnet, daß als Radikalinitiatoren Verbindungen eingesetzt werden, die mindestens drei Hydroperoxid-Einheiten, Peroxid-Einheiten oder Azo-Einheiten enthalten.

6. Wasserquellbare hydrophile Polymerzusammensetzung gemäß einem oder mehreren der Ansprüche 1 bis 5, dadurch gekennzeichnet, daß als Radikalinitiator ein Umsetzungsprodukt von Azobisisobutyronitril mit Trimethylolpropan eingesetzt wird.

7. Wasserquellbare hydrophile Polymerzusammensetzung gemäß einem oder mehreren der Ansprüche 1 bis 5, dadurch gekennzeichnet, daß als Radikalinitiator ein durch anodische Oxidation einer Polycarbonsäure in Gegenwart von Sauerstoff erhaltenes Polyhydroperoxid eingesetzt wird.

8. Wasserquellbare hydrophile Polymerzusammensetzung gemäß einem oder mehreren der Ansprüche 1 bis 7, dadurch gekennzeichnet, daß sie unter Verwendung eines Vernetzers hergestellt wird.

9. Verfahren zur Herstellung der wasserquellbaren hydrophilen Polymerzusammensetzungen gemäß einem oder mehreren der Ansprüche 1 bis 8, dadurch gekennzeichnet, daß eine 15 bis 60 Gew.-%ige wäßrige Lösung eines oder mehrerer hydrophiler Monomerer in Anwesenheit von Stärke und/oder chemisch modifizirter Stärke nach dem Verfahren der Gelpolymerisation in Gegenwart eines Radikalinitiators, der Tri- oder Polyradikale bilden kann, polymerisiert wird.

10. Verwendung der wasserquellbaren hydrophilen Polymerzusammensetzungen gemäß einem oder mehreren der Ansprüche 1 bis 8 als Absorptionsmittel für Wasser und wäßrige Flüssigkeiten in Hygieneartikeln.
